# EUROPEAN PATENT SPECIFICATION

(11) **EP 1 617 867 B1**
(45) Date of publication and mention of the grant of the patent: **26.10.2011**
(21) Application number: 03723085.1
(22) Date of filing: 30.04.2003
(51) Int. Cl.: A61K 39/39, A61K 35/74, A61P 37/04, A61P 37/06

(54) **IMMUNOMODULATING COMPOSITION COMPRISING A PARTICULATE FRACTION OF BACTERIAL MECHANICAL LYSATES**
IMMUNOMODULIERENDE ZUSAMMENSETZUNG DIE EINE PARTIKELFRAKTION VON BAKTERIELLEN MECHANISCHEN LYSATES ENTHÄLT
COMPOSITION IMMUNOMODULATRICE CONTENANT UNE FRACTION DE PARTICULES DE LYSATS MECANIQUES BACTERIENS

(43) Date of publication of application: 25.01.2006
(73) Proprietor: Medi Service S.R.L., 20041 Agrate Brianza (IT)
(72) Inventor: MELIOLI, Giovanni, I-16125 Genova (IT); FASANI, Roberto, I-24122 Bergamo (IT)
(74) Representative: Germinario, Claudio
(86) International application number: PCT/IT2003/000267
(87) International publication number: WO 2004/096270

(56) References cited:
- FR-A- 2 307 542
- FR-A- 2 822 071
- ZELLE-RIESER CLAUDIA ET AL: "A clinically approved oral vaccine against pneumotropic bacteria induces the terminal maturation of CD83+ immunostimulatory dendritic cells" IMMUNOLOGY LETTERS, vol. 76, no. 1, 1 February 2001 (2001-02-01), pages 63-67, XP001188266 ISSN: 0165-2478
- NAKAHARA SAORI ET AL: "Efficient generation of mature dendritic cells using Streptococcal preparation (OK-432)" PROCEEDINGS OF THE AMERICAN ASSOCIATION FOR CANCER RESEARCH ANNUAL, vol. 42, March 2001 (2001-03), pages 25-26, XP008027799 92nd Annual Meeting of the American Association for Cancer Research;New Orleans, LA, USA; March 24-28, 2001, March, 2001 ISSN: 0197-016X
- BERER ANDREA ET AL: "1,25-Dihydroxyvitamin D3 inhibits dendritic cell differentiation and maturation in vitro" EXPERIMENTAL HEMATOLOGY (CHARLOTTESVILLE), vol. 28, no. 5, May 2000 (2000-05), pages 575-583, XP002271634 ISSN: 0301-472X
- BOUREAU H ET AL: "PROTEIN CONTENTS OF BACTERIAL LYSATES OBTAINED BY DIFFERENT PROCEDURES" ANNALES PHARMACEUTIQUES FRANCAISES, vol. 37, no. 9-10, 1979, pages 357-368, XP008027741 ISSN: 0003-4509
- SOLTENSZKY J ET AL: "THE HOSTS' DEFENSE INCREASING ACTIVITY OF AUTOLYSATE COMBINATION PREPARED FROM SERRATIA-MARCESCENS AND STAPHYLOCOCCUS-AUREUS STRAINS" ANNALES IMMUNOLOGIAE HUNGARICAE, vol. 26, no. 1, 1986, pages 339-344, XP008027737 ISSN: 0570-1708
- TOYOKAWA HIDEYOSHI ET AL: "Enhancement of circulating dendritic cell activity by immunomodulators (OK432 and KP-40)" ANTICANCER RESEARCH, vol. 22, no. 4, July 2002 (2002-07), pages 2137-2146, XP008027771 ISSN: 0250-7005
- KEKOW J ET AL: "POLYCLONAL B-CELL ACTIVATION BY IMMUNO-AUGMENTATION" DMW (DEUTSCHE MEDIZINISCHE WOCHENSCHRIFT), vol. 114, no. 28-29, 1989, pages 1101-1106, XP000802777 ISSN: 0012-0472

## Description

### Field of the invention

The present invention relates to bacterial antigenic preparations and pharmaceutical immunocompositions containing particulate fractions of bacterial mechanical lysates and methods for their preparation.

### State of the art

Immunomodulating compositions of different type are known in the state of the art e.g. compositions containing LPS endotoxines, or Bacillus Calmette Guerin (BCG) that is an attenuated strain of Mycobacterium tuberculosis, or Double Strand RNAs (DS-RNA) or, finally, immunomodulating compositions containing bacterial lysates. The drawbacks of the first three kinds of compositions are directly related to safety, in fact, LPS can be used only in vitro; BCG, although used in human therapy has caused several cases of an infective disease called BGCitis and DS-RNA cannot be used on humans due to its high potentiality of toxicity and oncogenicity.

Soluble bacterial lysates obtained by alkaline treatment such as the commercial product Broncovaxon^{(R)} are also known in the state of the art. Bacterial compositions of this art, lack of intact molecules capable of interaction with the TOLL-like receptors expressed on macrophage cells and, hence, on dendritic precursor cells. In fact, maturation of the dendritic cells also depends on the interaction of said receptors with superficial structures of pathogenic bacteria.

Particulate immunomodulating compositions are also known in the state of the art. These are polyvalent bacterial compositions containing the particulate fraction of mechanical lysates of different bacterial strains. Commercial products such as Ismigen® Immubron® are available, which comprise a mixture of debris obtained from the mechanical fragmentation of the specific bacteria Staphylococcus aureus, Klebsiella Pneumoniae - ozaenae, Diplococcus pneumoniae, Haemophylous influenzae, Streptococcus pyogenes - viridans, Neisseria catarrhalis. FR. 2307542A discloses an immunostimulating composition comprising mechanical lysates of 6 bacteria. In the most recent terminology, Diplococcus pneumoniae and Neisseria catarrhalis are also designated as Streptococcus pneumoniae and Moraxella catarrhalis respectively.

However, compositions comprising material from so many bacterial strains are difficult to produce and are not free of problems in relation to standardisation of the production, standardisation of the regimen of administration and the consistency of the obtained effects. Moreover, although the known compounds already exhibit a considerable immunostimulating activity, they have a defined predetermined activity, which does not allow any flexibility in the adaptation of the effect to specific clinical necessities. The state of the art, hence, leaves several open needs related to the above mentioned type of immunocompositions; among them, the need of providing compositions that can be produced and standardized more easily; and the need of providing even more efficient immunomodulating products capable either of stimulating or of inhibiting the immunoresponse, but mainly able to be adapted to any specific clinical necessity, thus suitable for a customised treatment.

### Summary of the invention

The present invention solves all the different aspects of the above-mentioned problems, by providing polyvalent compositions comprising the particulate fractions from lysates obtained by mechanical lysis of different bacterial species a method for selecting said bacterial species and a method of preparing said compositions. These compositions exhibit immunostimulation or immunomodulation activity, in the meaning of inducing the maturation of immature dendritic cells.

Hence, object of the present invention is a method of selecting bacteria or combination of bacteria suitable for preparing immunocompositions comprising the particulate fraction of lysates obtained by mechanical lysis of such bacteria for patient-customized treatment comprising the steps of:
selecting bacteria or combination of bacteria from the following genera *Staphylococcus, Klebsiella, Diplococcus, Haemophylous, Streptococcus, Neisseria,*
determining the expression level of the four membrane markers DC80, DC83, DC86 and HLA-II induced by each bacterium or combination of bacteria in immature dendritic cells;
calculating for each bacterium or combination of bacteria a cumulative score that is the sum of the amounts of the four expressed markers,
listing all bacteria or combination of bacteria by increasing cumulative score, wherein the higher the cumulative scores the higher the immunostimulation effect.

A second object of the invention is the immunocomposition for use in a medical immunetreatment, comprising the particulate fraction of lysates obtained by mechanical lysis of the selected bacteria or combinations of bacteria. The immunocomposition stimulating the maturation of immature dendritic cells comprising the particulate fraction of lysates obtained by mechanical lysis of bacteria from five or less genera selected from the group consisting of *Staphylococcus, Klebsiella, Diplococcus, Haemophylous, Streptococcus, Neisseria* and wherein the bacteria are selected from *Staphylococcus aureus, Klebsiella spp, Diplococcus pneumoniae, Haemophylous influenzae, Streptococcus spp, and Neisseria catarrhalis*. For Klebsiella spp is meant K. pneumoniae and/or ozaenae, for Streptococcus spp is meant S. pyogenes.and/or viridans.

Advantageously, the composition of the invention, may comprise at least one of the species *Klebsiella* spp, *Haemophylous influenzae* and *Neisseria catarrhalis* and induces the complete maturation of the immature dendritic cells, with complete expression of costimulating molecules CD83, CD80, CD86 and/or HLA class II.

Different realisation forms of the invention entail polyvalent compositions comprising specific combinations of two, three, four or five members from *Staphylococcus* aureus, *Klebsiella spp, Diplococcus pneumoniae, Haemophylous* influenza, *Streptococcus spp, Neisseria catarrhalis*.

A further object of the present invention is a method for preparing a particulate immunocomposition, comprising the selected bacteria or combination of bacteria wherein bacterial culture cells selected from Staphylococcus aureus, Klebsiella spp, Diplococcus pneumoniae, Haemophylous influenzae, Streptococcus spp, Neisseria catarrhalis are mechanically lysed, then the particulate fractions of the lysates are collected and optionally freeze-dried. The lyophilized or suspended antigenic material may be formulated into suitable pharmaceutical compositions. Depending on the selected bacteria, compositions are prepared, which cause induction of a complete maturation of the immature dendritic cells. When the composition comprises material from two, three, four or five bacterial cultures, the corresponding lysates are individually prepared and then mixed to obtain the desired activity.

A still further object of the invention is the composition in suit for use in a medical immunotreatment, advantageously, in a patient customized treatment.

The above-described composition is administered in a therapeutically effective dose, possibly patient-adapted dose to obtain the necessary effect.

The compositions of the invention offer indisputable advantages. They can be produced, and their medical effect standardized, more easily and efficiently than those of the prior art, due to the lower number of strains used. Moreover, depending on the type of selected bacteria and specific combinations, these compositions are capable of modulating the immunoresponse of the patient. This property offers ample possibilities of adapting the compositions and the treatment to any specific clinical necessity, thus enabling the realisation of patient customised treatments.

A further advantage of the compositions of the invention is that they may exhibit the same immunostimulating activity of the known compositions or even a much higher immunostimulating activity while containing material from a lower number of bacterial genera.

### Description of the figures

Figure 1 is the graphic representation of the scores calculated for different combinations of bacteria. The scores are the sum of the amounts, determined by immunofluorescence, of the four membrane markers CD83, CD86, CD80 and HLA-II expressed by all the bacteria species of each combination. All the combinations are gathered in four clusters.
Figure 2 is a graphic representation of the scores by cluster 1.
Figure 3 is a graphic representation of the scores by cluster 2.
Figure 4 is a graphic representation of the scores by cluster 3.
Figure 5 is a graphic representation of the scores by cluster 4.
Figure 6 illustrates a scatter plot: Distances from the cluster centre vs Score for each case.
Figure 7 illustrates the average score by cluster.

### Detailed description of the invention

Dendritic cells are specialize antigen-presenting cells. Their maturation is a necessary first step for induction of adaptive immunity. The function of dendritic cells is to carry pathogen antigens to peripheral lymphoid organs and there to present them to T-lymphocytes. When a dendritic cell takes up a pathogen in infected tissue, it becomes activated and travels to a nearby lymph node. On activation, the dendritic cell mature into a highly efficient antigen-presenting cell (APC) and undergoes changes that enable it to activate pathogen specific lymphocytes in the lymph nodes. Immunoresponse through dendritic cells needs several costimulating molecules, namely CD80, CD83 and CD86. These are conjugated with the respective ligands on the effectors lymphocytes to which the relevant peptide is presented through class I or class II HLA - the higher the expression of co-stimulatory molecules on the surface of dendritic cells, the more efficient the capability of presenting the antigen and recruiting effector lymphocytes. The final effect is an enhancement of the immunoresponse, thus immunostimulation. On reverse, a lower expression of co-stimulatory molecules will result in a lower effectiveness in the antigen presentation.

The particulate material according to the invention is able to perform both the above-indicated functions, depending on the selected bacteria and/or bacterial combinations. In particular, the stimulation of complete maturation of immature dendritic cells is accompanied by the expression not only of CD83, but also of CD80 and/or CD86 and/or HLA class II.

Particulate or corpuscolate fractions within the meaning of the present invention are bacterial lysate fractions containing insoluble particulate antigens. Antigens in this form cannot be filtered or centrifuged at high speed without undergoing significant modifications of their immunological characteristics. Unlike particulate antigen, the soluble antigen known in the prior art once solubilized in any suitable solvent can be filtered or centrifuged at high speed without their immunological characteristics being affected.

Another important feature of the invention is that the preparation of the particulate fractions does not involve drastic solubilisation steps, as it is the case for the soluble antigens known in the prior art. For this reason, the antigenic proteins of the invention maintain all its native structures, i.e. primary, secondary, tertiary and quaternary structures, virtually intact. As a consequence, an antibody response elicited by a particulate antigenic fraction is not only directed to simple antigens resulting from the primary and secondary structures, but also to complex or repeated antigens resulting from the primary, secondary, tertiary or quaternary structures. This is very important for the efficiency of the antibody immunoresponse, that is mainly directed (about 80%) to three-dimensional conformational antigenic sites.

There also exists a dramatic difference in the action-mechanism between the soluble antigen of the prior art and the antigen of the invention. The response caused by corpuscolate antigens is mediated by the cytotoxic activity of CD4 and CD8. This results in a much broader spectrum of antibody response. Finally, the activation of dendritic cells, which is the starting point of the cell maturation and antigen presentation processes, mediates the co-stimulation of many other cells contributing to the complete immunoresponse. This activation is efficiently performed by particulate antigens, but very poorly by soluble antigens. The activation is in fact mediated by Toll-like receptors, which are specific for polysaccharide structures, such as protidoglycans and lipopolysaccharides, which are peculiar of the intact bacterial wall. A still further effect is that the immunoresponse to the bacterial wall fragments is accompanied by an efficient opsonisation activity not present in the case of soluble antigens.

The ability of particulate antigens to trigger a highly efficient immunoresponse makes it possible to prepare immunocompositions free of any sort of immunoadjuvants such as Mycobacterium tuberculosis, mineral oils, or any other additive potentially harmful for human health, but usually present in antigenic compositions comprising soluble antigens.

The particulate fraction of bacterial lysate of the invention is obtained by mechanical lyse. Any known physical method suitable to fragment bacterial cells, and to produce membrane debris with unaffected antigenic structures may be used. Suitable means are press, highpressure valve, ball mill, grinding, sonication, osmosis, cryolysis (freezing and thawing). The particulate insoluble fraction of the bacterial lysate is then subjected to separation from the soluble components of the lysate. The step is carried out according to known techniques suitable for the separation of insoluble phases, such as washing, filtration, centrifugation or combination of these techniques.

The preparation process of the antigenic insoluble fraction may comprise anyone of the following procedural steps:
a. Identification of the selected bacteria species;
b. Culture and propagation in growth medium of the selected strains;
c. Collection from the culture medium of the bacterial cells and washing;
d. Fixation with formaldehyde or heat inactivation;
e. Mechanical lysis of the bacterial cells;
f. Washing of the particulate fraction and elimination of the soluble fraction;
g. Freeze-drying of the particulate fraction comprising the cell debris;
h. Mixing of freeze-drying material from two or more selected species to produce the desired antigenic combinations.

It is plain that, with the exception of the mechanical lysis and the isolation of the insoluble fraction, all the remaining steps are optional and can be carried out in any practically acceptable procedural order and according to any suitable known technique.

The so obtained insoluble fractions, in the form of a liquid suspension or a solid freeze-dried material, are formulated.into a pharmaceutical composition suitable for human or animal administration.

These compositions are sterile or not sterile liquid formulations, in form of drops, micronized drops, spray, and aerosol, for oral, intranasal or in general mucosal administration. Sterile suspension can be used for endovesical, endothoracic, endoperitoneal, hypodermal, intramuscular injection. The solid freeze-dried material may be formulated into solid pharmaceutical forms such as tablets, pills, or lozenges. Preferred forms are tablets or lozenges for sublingual administration. Alternatively, the liquid suspension is freeze-dried in vials, in monodose amounts suitable for reconstitution with sterile liquid medium on need. When the compositions of the invention are used as an aspecific adjuvant for specific immunisation, they may comprise, beyond the particulate antigens of the invention, any other desired antigens or allergens.

The amounts of the particulate antigenic material and the selections of specific combinations of bacteria depend on the need and conditions of the patient. When this is used as an adjuvant for aspecific immunostimulation, the amount ranges from 0.1 µg/day to 10 mg/day, preferably from 10 µg/day to 1 mg/day, or about 0,5 mg/day. When used for specific immunostimulation, the amount ranges from 100 µg/day to 10 mg/day, preferably from 1 mg/day to 5 mg/day. When used for immunomodulation, the amount ranges from 0.1 µg/day to 10 mg/day, preferably from 10 µg/day to 1 mg/day. All the aforementioned amounts are independent from the administering route.

The monodose formulations comprise the same daily amounts or a submultiple of the same. The compositions of the invention may also optionally comprise any pharmaceutically acceptable carrier, excipient or additive typical of the immunocompositions.

Prefer red bacteria or combinations of bacteria are listed in Table 1 below along with the commercial product Ismigen. Table 1 also reports for each bacterium or combination of bacteria a score reflecting the specific capability of inducing maturation of the immature dendritic cells

**Table 1**

| | | | | | | |
|---|---|---|---|---|---|---|
| S aureus | Streptococcus spp | | | | | 23,3 |
| Saureus | | | | | | 24,0 |
| Streptococcus spp | | | | | | 102,0 |
| D pneumoniae | Streptococcus spp | | | | | 131,8 |
| D pneumoniae | | | | | | 140,00 |
| S aureus | D pneumoniae | | | | | 153,6 |
| D Pneumoniae | H influenzae | Streptococcus spp | | | | 190,2 |
| Streptococcus spp | | | | | | |
| S aureus | H influenzae | | | | | 203,0 |
| S aureus | H Influenzae | Streptococcus spp | | | | 203,6 |
| H Influenzae | Streptococcus spp | | | | | 204,9 |
| Klebsiella spp | H influenzae | | | | | 224,2 |
| S aureus | Klebsiella spp | | | | | 224,5 |
| Klebsiella spp | H influenzae | Streptococcus spp | | | | 232,3 |
| S aureus | Klebsiella spp | Streptococcus spp | | | | 260,5 |
| S aureus | D pneumoniae | Streptococcus spp | | | | 268,5 |
| Klebsiella spp | Streptococcus spp | M catarrhalis | | | | 280,6 |
| S aureus | Klebsiella spp | M catarrhalis | | | | 285,6 |
| Klebsiella spp | D pneumoniae | Streptococcus spp | M catarrhalis | | | 287,6 |
| H Influenzae | | | | | | 290,0 |
| Klebsiella spp | H Influenzae | M catarrhalis | | | | 302,0 |
| S aureus | Klebsiella spp | D pneumoniae | | | | 305,5 |
| S aureus | H Inftuenzae | M catarrhalis | | | | 312,9 |
| Klebsiella spp | Streptococcus spp | | | | | 334,9 |
| S aureus | H Influenzae | Streptococcus spp | M catarrhalis | | | 336,9 |
| Klebsiella spp | D pneumoniae | | | | | 346,8 |
| Klebsiella spp | D pneumoniae | H Influenzas | M catarrhalis | | | 350,4 |
| H influenzas | Streptococcus spp | M catarrhalis | | | | 350_{:}9 |
| D pneumoniae | H influenzae | M catarrhalis | | | | 361,5 |
| S aureus | Klebsiella spp | H influenzae | M catarrhalis | | | 371,1 |
| S aureus | Klebsiella spp | D pneumoniae | H influenzae | Streptococcus spp | | 372,5 |
| S aureus | Klebsiella spp | D pneumoniae | Streptococcus spp | | | 375,0 |
| Klebsiella spp | D pneumoniae | H Influenzae | Streptococcus spp | M catarrhalis | | 377,0 |
| S aureus | D pneumoniae | H influenzae | Streptococcus spp | | 377,4 | 377,4 |
| S aureus | Klebsiella spp | Streptococcus spp | M catarrhalis | | | 378,2 |
| H Influenzae | M catarrhalis | | | | 383,4 | 383,4 |
| D pneumoniae | H Influenzae | Streptococcus spp | M catarrhalis | | | 387,1 |
| S aureus | Klebsiella spp | H influenzae | | | | 388,8 |
| S aureus | Klebsiella spp | D pneumoniae | H influenzae | | | 389,4 |
| Klebsiella spp | D pneumoniae | H Influenzae | Streptococcus spp | | | 396,6 |
| S aureus | Streptococcus spp | M catarrhalis | | | | 398,2 |
| Klebsiella spp | M catarrhalis | | | | | 399,9 |
| S aureus | Klebsiella spp | D pneumoniae | H influenzae | Streptococcus spp | M catarrhalis | 400,0 |
| S aureus | D pneumoniae | H influenzae | M catarrhalis | | | 403.4 |
| S aureus | Klebsiella spp | D pneumoniae | H Influenzae | M catarrhalis | | 410,0 |
| Klebsiella spp | D pneumoniae | M catarrhalis | | | | 414,1 |
| Klebsiella spp | H influenzae | Streptococcus spp | M catarrhalis | | | 414,8 |
| D pneumoniae | Streptococcus spp | M catarrhalis | | | | 416,8 |
| S aureus | D pneumoniae | M catarrhalis | | | | 419,2 |
| S aureus | Klebsiella spp | H Influenzas | Streptococcus spp | spp | | 420,3 |
| S aureus | D pneumoniae | Streptococcus spp | M catarrhalis | | | 425,2 |
| S aureus | Klebsiella spp | H Influenzae | Streptococcus spp | M catarrhalis | | 432,8 |
| S aureus | Klebsielia spp | D pneumoniae | M catarrhalis | | | 441,7 |
| Klebsiella spp | D pneumoniae | Streptococcus spp | | | | 447,9 |
| D pneumoniae | S aureus | H influenzae | Streptococcus spp | M catarrhalis | | 450,4 |
| Klebsiella spp | D pneumoniae | H influenzae | | | | 450,5 |
| Klebsiella spp | | | | | | 458,0 |
| M catarrhalis | | | | | | 463,0 |
| D pneumoniae | H influenzae | | | | | 470,7 |
| S aureus | D pneumoniae | H Influenzae | | | | 476,6 |
| Streptococcus spp | M catarrhalis | | | | | 488,8 |
| D pneumoniae | M catarrhalis | | | | | 531,0 |
| S aureus | M catarrhalis | | | | | 533,4 |

The scores, which range from 23,3 to 533,4, are the summation of the amounts of the four membrane markers, CD83, CD86, CD80 and HLA-II expressed by all the bacteria species of each combination. The amount of expression of the markers is determined by immunofluorescence as described in the experimental section.

The score obtained by the commercial product Ismigen (S.aureus, Klebsiella spp, S.pneumoniae, H.influenzae, Streptococcus spp, M catarrhalis), is 400, and is taken as reference value. All the scores higher than 400 identify an improved induction of the maturation of immature dendritic cells as regard to the reference value and an aspecific immunostimulation, whereas scores lower than 400 identify a lower induction of dendritic cell maturations as regard to the reference value. All the observed scores have been processed according to the biometrical method described in the experimental section, and gathered in four clusters labelled as cluster 1 to 4. The bacteria and bacteria combinations comprised in each cluster exhibit different immunoactivity. In particular, it was experimentally observed that the presence of insoluble antigenic material from *Klebsiella spp*, *Haemophylous influenzae* or *Neisseria catarrhalis* enhanced the capability of complete maturation of immature dendritic cells, and results in immunostimulation. On the contrary, the presence of insoluble antigenic material from *Staphylococcus* aureus, *Diplococcus* pneumoniae or *Streptococcus ssp* (*pyogenes and*/*or viridans*) induces a lower maturation of immature dendritic cells. This latter observation, i.e. immunosuppression, is extremely interesting since it represents the first evidence that there exist bacteria capable of inhibiting the immunoresponse through an escape mechanism so far unknown for bacteria. Actually, only herpes virus was known to inhibit the immunoresponse through a down regulation of the expression of CD83 after infection.

The compositions of the invention may be used in specific or aspecific therapeutic treatments, including prevention or adjuvation, against bacteria infections, in particular of respiratory infections. By accurate selection of the specific bacteria or combination of bacteria it is also possible to adapt the immunocompositions to the specific conditions and needs of the patient to be treated, so enabling a patient customized treatment to be devised.

The immunostimulating compositions find application in all those normal or pathological conditions in which the induction or stimulation of the complete maturation of immature dendritic cells may be useful or necessary. Thus the compositions in suit are used to achieve the effect of aspecific stimulation of the immunosystem; aspecific immunopotentiation upon vaccination; specific immunoprophylaxis for infections caused by bacterial strains contained in the composition; immunopotentiation of a vaccine effect in "non responder" patients; aspecific immunotherapy of tumours such as melanoma, bladder superficial tumour, NSCLC; specific immunotherapy of tumours with tumour-associated antigens of different molecular weight or tumour-associated lymphoid effectors or loaded dendritic cells; activation of the macrophage system as a scavenger in chronic diseases, decubitus or phagedenic ulcers, and tumours of the craniocephalic district.

The immunomodulating compositions of the invention also find application in the treatment and prevention of autoimmuno diseases, allergy, graft versus host disease and transplant rejection.

The present invention also encompasses the therapeutic treatment of patients in need. The treatment implies the daily administration of therapeutically active doses of the particulate antigenic material, for 1 to several days in case of prophylactic treatment, or as long as the condition to be treated persists.

The invention is further described in all details by way of examples of experimental methods, which, however, have no limiting effect on the scope of protection.

### Preparation of the antigenic particulate fraction.

The mechanical lysates of typical respiratory-infection bacteria (Diplococcus pneumoniae, Streptococcus pyogenes, Staphylococcus aureus, Klebsiella pneumoniae, Haemophylous influenzae, Neisseria (Moraxella) catarrhalis) were obtained as follows. Living bacteria for in vitro studies were obtained from available bacterial collections. The purity of the bulk culture was checked using clinical microbiology routine identification procedures. After in vitro expansion of each strain, bacteria were extensively washed, then subjected to a mechanical fragmentation using a highpressure valve. Following a further washing step, the powder was lyophilized and stored at 4° C. Microscopic examination of fragmented bacteria after Gram staining documented the presence of bacterial debris maintaining the original Gram characteristics.

The antigenicity of these preparations was checked using rocket electrophoresis against a panel of polyclonal anti-SA, anti Spy, anti-KP, anti-HI, anti-MC and anti-Spn, obtained in rabbits according to known protocols. To confirm the immunological activity of these reagents, the particulate antigens were partially dissolved using overnight agitation with 0.2% Tween 20 in phosphate buffered saline and the non-particulate fraction was analyzed using rocket electrophoresis.

### Isolation and culture of human dendritic cells

human dendritic cells were derived from human peripheral blood mononuclear cells according to known a method. Briefly, highly purified monocytes were treated with GM-CSF and IL-4 for 5 days then their maturation was induced using different concentration of the PMBL. As controls, TNF-a, LPS, BCG were employed.

### Immunofluorescence method

The determination of the expression level of cell-markers CD83, CD86, CD80 and HLA-II was carried out by way of the following immunofluorescence method:
Dendritic cells are treated with a monoclonal antibody specific for the CD83 antigen. Other cells are treated with monoclonal antibodies to CD80, CD86 and HLA class II. The concentration of each antibody is set a priori to a specific title. All antibodies are labelled with a fluorochrome molecule such as fluorescin diacetate. After 30 min, the cells are washed with 2 ml of saline solution, centrifuged and suspended again in a small volume of saline solution. Then, the cells are analyzed by flow cytometry, a technique enabling the evaluation of the percentage of the cells expressing one defined antigen from the whole pool of the analyzed cells.

The technique also allows the determination of the average number of antigen molecules expressed on the cell membrane, since this number is proportional to fluorescence intensity measured by the cyto-fluorimeter. In this way, it is possible to assess the fluorescence intensity on immature dendritic cells, which is extremely low since the aforementioned antibodies only recognize antigens expressed during the maturation of the same cells, and the intensity of mature dendritic cells, which on the contrary is very high. The evaluation of the fluorescence intensity in different experimental conditions provides evidence whether such a condition inhibits or induces a complete maturation of dendritic cells. This appears when either very low or extremely high fluorescence is respectively detected on the cells.

### Biometrical elaboration of the results.

The immunological activity of the following six bacterial species and that of many of their possible combinations are evaluated:
A STAPHYLOCOCCUS AUREUS
B KLEBSIELLA PNEUMONIE - OZAENAE
C DIPLOCOCCUS PNEUMONIE
D HAEMOPHILUS INFLUENZAE
E STREPTOCOCCUS PYOGENES - VIRIDANS
F NEISSERIA CATARRHALIS

For each bacterial species, the following four membrane markers are considered: HLA class II, CD80, CD83 and CD86.

Single bacteria or arbitrary combination of bacteria have been selected and for each selection/combination the sum of the amounts of the four expressed markers is calculated according to the above described fluorimetric method. Cumulative scores are then obtained, which depends on the level of marker expression. This score is set to 400 for the reference combination of six bacteria known in the prior art (Ismigen).

The so obtained results are gathered in four clusters, according to the biometrical method of Cluster Analysis, on the basis of their homogeneity calculated in terms of distribution around the cluster centre. Operatively, the relative distance between the score of each combination to the centre of the cluster is calculated with the method of K-average value. To better evaluate the relative distances, the method of iterative adaptation of the cluster-centres was adopted. Finally, the correct association of any combination to one specific cluster was confirmed through Fischer analysis of the statistical variances.

The results are reported in Table 2 below, in which the distance from the cluster centre for each bacterium or combination of bacteria is reported, along to the number (1 to 4) of the cluster to which each member is attributed.

**Table 2**

| Bacteria | Cluster | Distance from cluster centre |
|---|---|---|
| AE | 1 | 72,483 |
| A | 1 | 71,783 |
| E | 1 | 6,217 |
| CE | 1 | 36,017 |
| C | 1 | 44,217 |
| AC | 1 | 57,817 |
| CDE | 3 | 60,865 |
| E | 3 | 59,065 |
| AD | 3 | 48,065 |
| ADE | 3 | 47,265 |
| DE | 3 | 46,165 |
| BD | 3 | 26,865 |
| AB | 3 | 26,565 |
| BDE | 3 | 18,765 |
| ABE | 3 | 9,435 |
| ACE | 3 | 17,435 |
| BEF | 3 | 29,535 |
| ABF | 3 | 34,535 |
| BCEF | 3 | 36,535 |
| D | 3 | 38,935 |
| BDF | 3 | 50,935 |
| ABC | 3 | 54,435 |
| ADF | 3 | 61,835 |
| BE | 2 | 50,352 |
| ADEF | 2 | 46,352 |
| BC | 2 | 38,452 |
| BCDF | 2 | 34,852 |
| DEF | 2 | 34,352 |
| CDF | 2 | 23,752 |
| ABDF | 2 | 14,152 |
| ABCDE | 2 | 12,752 |
| ABCE | 2 | 10,252 |
| BCDEF | 2 | 8,252 |
| ACDE | 2 | 7,852 |
| ABEF | 2 | 7,052 |
| DF | 2 | 1,852 |
| CDEF | 2 | 1,848 |
| ABD | 2 | 3,548 |
| ABCD | 2 | 4,148 |
| BCDE | 2 | 11,348 |
| AEF | 2 | 12,948 |
| BF | 2 | 14,648 |
| ACDF | 2 | 18,148 |
| ABCDF | 2 | 24,748 |
| BCF | 2 | 28,848 |
| BDEF | 2 | 29,548 |
| CEF | 2 | 31,548 |
| ACF | 2 | 33,948 |
| ABDE | 2 | 35,048 |
| ACEF | 2 | 39,948 |
| ABDEF | 4 | 37,600 |
| ABCF | 4 | 28,700 |
| BCE | 4 | 22,500 |
| CADEF | 4 | 20,000 |
| BCD | 4 | 19,900 |
| B | 4 | 12,400 |
| F | 4 | 7,400 |
| CD | 4 | ,300 |
| ACD | 4 | 6,200 |
| EF | 4 | 18,400 |
| CF | 4 | 60,600 |
| AF | 4 | 63,000 |

### Graphical representation of immunoresponse triggered by the different clusters:

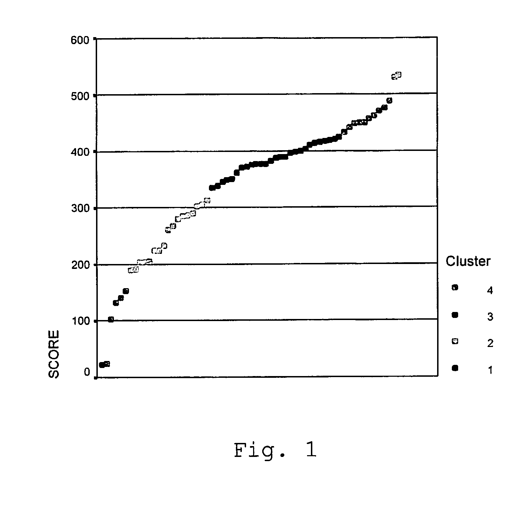

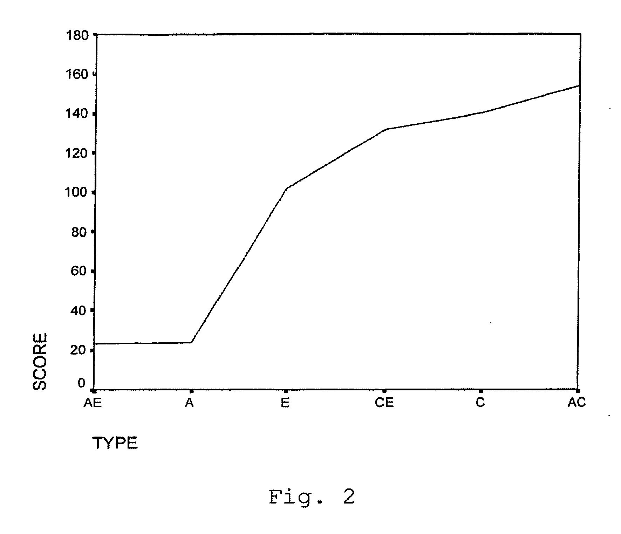

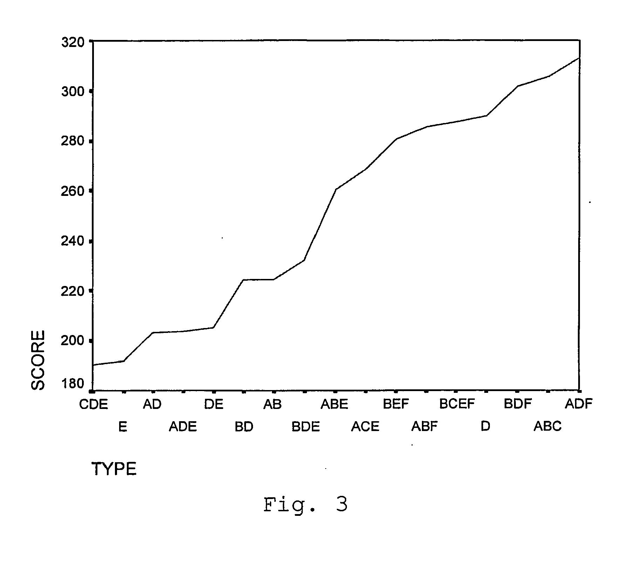

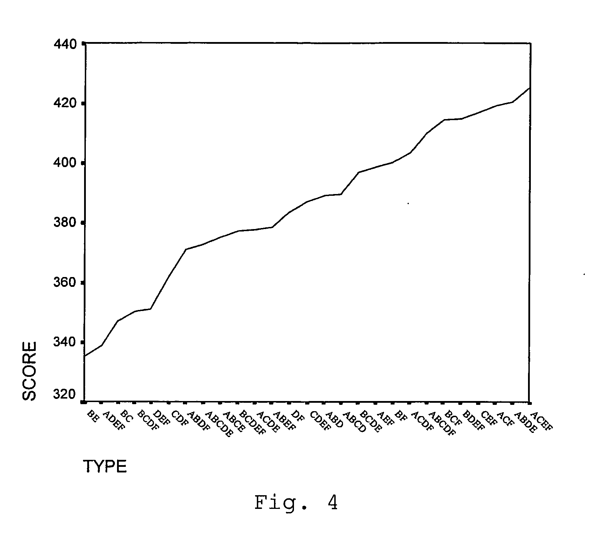

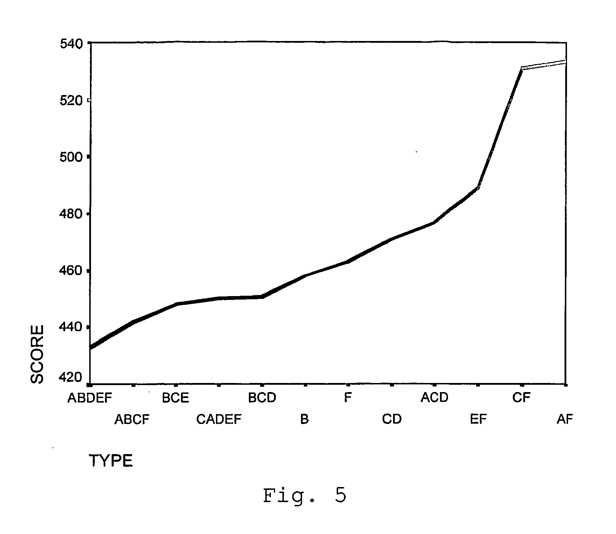

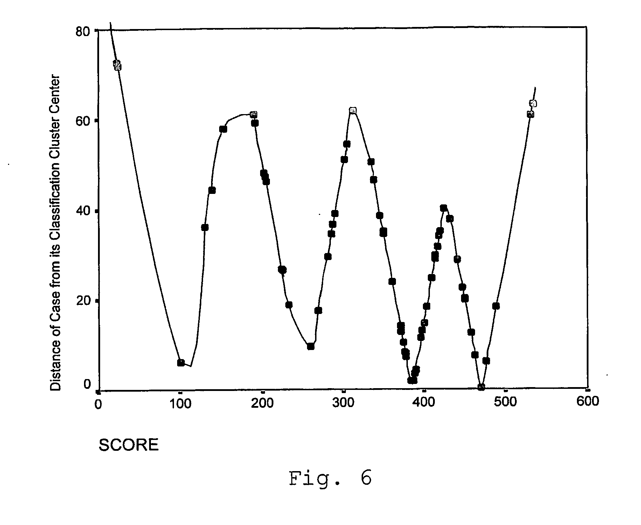

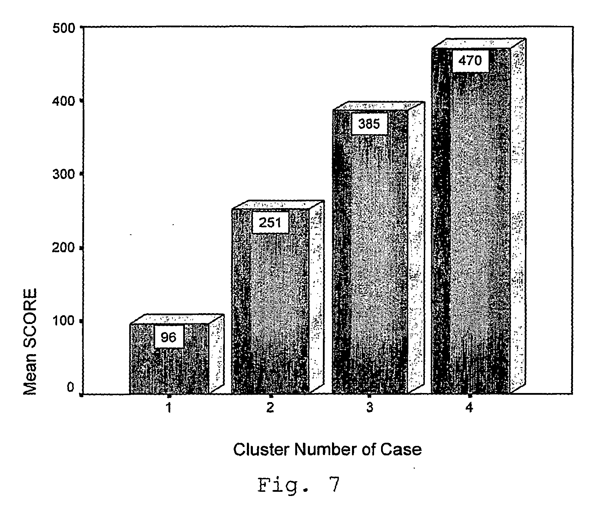

The bacteria or bacterial combinations belonging to the different clusters are experimentally evaluated as to their cumulative level of expression of the four membrane markers. The members of cluster 4 show the most efficient expression of membrane markers, as evident from the corresponding scores, which are always higher than the reference value 400, attributed to the known product (figures 1 to 7). The bacteria or bacterial combinations of this cluster do elicit the highest immunostimulating response.

### Contribution due to each factor of cluster 4

The contribution, in terms of percent increase or decrease of the score, due to the different factors (bacteria or combinations, of bacteria) belonging to cluster number 4 has been evaluated according to the following method.

The score obtained with a reference bacterium taken alone is arbitrary set on 100. Then the score obtained by combinations comprising said reference bacterium is assessed.

| ***F (NEISSERIA CATARRHALIS)* :** 100 | | | |
|---|---|---|---|
| **Added** | A. | **Obtained** | 115 |
| | B | | 86 |
| | C | | 115 |
| | D | | 83 |
| | E. | | 105 |
| **Added** | AB | **Obtained** | 62 |
| | AC | | 90 |
| | AD | | 68 |
| | AE | | 86 |
| | BC | | 89 |
| | BD | | 65 |
| | BE | | 61 |
| | CD | | 78 |
| | CE | | 90 |
| | DE | | 76 |
| **Added** | ABC | **Obtained** | 95 |
| | ABD | | 80 |
| | ABE | | 82 |
| | ACD | | 87 |
| | ACE | | 92 |
| | ADE | | 73 |
| | BCD | | 76 |
| | BCE | | 62 |
| | BDE | | 90 |
| **Added** | ABCD | **Obtained** | 89 |
| | ACDE | | 97 |
| | ABDE | | 94 |
| | BCDE | | 81 |

| ***B (KLEBSIELLA PNEUMONIE - OZAENAE)* :** 100 | | | |
|---|---|---|---|
| **Added** | A | **Obtained** | 49 |
| | C | | 76 |
| | D | | 49 |
| | E | | 73 |
| **Added** | AC | **Obtained** | 67 |
| | AD | | 85 |
| | AE | | 60 |
| | CD | | 98 |
| | CE | | 98 |
| | DE | | 51 |
| **Added** | ACE | **Obtained** | 82 |
| | ACD | | 85 |
| | ADE | | 92 |
| | CDE | | 87 |
| **Added** | ACDE | **Obtained** | 81 |

| ***C (DIPLOCOCCUS PNEUMONIE)* :** 100 | | | |
|---|---|---|---|
| **Added** | A | **Obtained** | 110 |
| | D | | 336 |
| | E | | 94 |
| **Added** | AD | **Obtained** | 341 |
| | DE | | 136 |
| | AE | | 192 |
| **Added** | ADE | **Obtained** | 269 |

The immunepotentiation or immunesuppression observed by single bacteria or combinations of bacteria is further highlighted in the following Table 3. The score of 400 of the commercial product Ismigen is taken as reference. Since this product is a combination of six members, the average contribution per member is about 66,7. The calculation of the real score observed for each bacterial species or bacterial combination of the invention shows a dramatic net difference as compared to the theoretical value resulting from the mere mathematical summation of the theoretical contributions due to each member of a combination, i.e. 66,7 per number of members. In particular the following bacteria or combination of bacteria result in a negative net effect:
*Staphylococcus aureus, Streptococcus spp (-110,0);*
*Staphylococcus aureus* (-42,7);
*Diplococcus (Staphyloc.) pneumoniae, Streptococcus spp* (-9,8);
*Diplococcus (Staphyloc.) pneumoniae, Streptococcus spp (-1,5)*.

On the contrary, all the other combinations give raise to a net positive effect, so indicating immunepotentiation.

**Table 3**

| | | | | | | | | |
|---|---|---|---|---|---|---|---|---|
| S aureus | Streptococcus spp | | | | | 23,3 | 2 | -110,0 |
| S aureus | | | | | | 24,0 | 1 | -42,7 |
| S pneumoniae | H influenzae | Streptococcus spp | | | | 190,2 | 3 | -9,8 |
| S pneumoniae | Streptococcus spp | | | | | 131,8 | 2 | -1,5 |
| S aureus | Klebsiella spp | S pneumoniae | H influenzae | Streptococcus spp | M catarrhalis | 400,0 | 6 | 0,0 |
| S aureus | H influenzae | Streptococcus spp | | | | 203,8 | 3 | 3,8 |
| S aureus | S pneumoniae | | | | | 153,6 | 2 | 20,3 |
| Klebsiella spp | S Pneumoniae | Streptococcus spp | M catarrhalis | | | 287,6 | 4 | 20,9 |
| Klebsiella spp | H influenzae | Streptococcus spp | | | | 232,3 | 3 | 32,3 |
| Streptococcus spp | | | | | | 102,0 | 1 | 35,3 |
| S aureus | Klebsiella spp | S pneumoniae | H influenzae | Streptococcus spp | | 372,5 | 5 | 39,2 |
| Klebsiella spp | S Pneumoniae | H influenzae | Streptococcus spp | M catarrhalis | | 377,0 | 5 | 43,7 |
| S aureus | Klebsiella spp | Streptococcus spp | | | | 260,5 | 3 | 60,5 |
| S aureus | S pneumoniae | Streptococcus spp | | | | 268,5 | 3 | 68,5 |
| S aureus | H influenzae | | | | | 203,0 | 2 | 69,7 |
| H influenzae | Streptococcus spp | | | | | 204,9 | 2 | 71,6 |
| S aureus | H influenzae | Streptococcus spp | M catarrhalis | | | 338,9 | 4 | 72,2 |
| S pneumoniae | | | | | | 140,0 | 1 | 73,3 |
| S aureus | Klebsiella spp | S pneumoniae | H influenzae | M catarrhalis | | 410,0 | 5 | 76,7 |
| Klebsiella spp | Streptococcus spp | M catarrhalis | | | | 280,6 | 3 | 80,6 |
| Klebsiella spp | S Pneumoniae | H influenzae | M catarrhalis | | | 350,4 | 4 | 83,7 |
| S aureus | Klebsiella spp | M catarrhalis | | | | 285,6 | 3 | 85,6 |
| Klebsiella spp | H influenzae | | | | | 224,2 | 2 | 90,9 |
| S aureus | Klebsiella spp | | | | | 224,5 | 2 | 91,2 |
| S aureus | Klebsiella spp | H influenzae | Streptococcus spp | M catarrhalis | | 432,8 | 5 | 99,5 |
| Klebsiella spp | H influenzae | M catarrhalis | | | | 302,0 | 3 | 102,0 |
| S aureus | Klebsiella spp | H influenzae | M catarrhalis | | | 371,1 | 4 | 104,4 |
| S aureus | Klebsiella spp | S pneumoniae | | | | 305,5 | 3 | 105,5 |
| S aureus | Klebsiella spp | S pneumoniae | Streptococcus spp | | | 375,0 | 4 | 108,3 |
| S aureus | S pneumoniae | H influenzae | Streptococcus spp | | | 377,4 | 4 | 110,7 |
| S aureus | Klebsiella spp | Streptococcus spp | M catarrhalis | | | 378,2 | 4 | 111,5 |
| S aureus | H influenzae | M catarrhalis | | | | 312,9 | 3 | 112,9 |
| S pneumoniae | S aureus | H influenzae | Streptococcus spp | M catarrhalis | | 450,4 | 5 | 117,1 |
| S pneumoniae | H influenzae | Streptococcus spp | M catarrhalis | | | 387,1 | 4 | 120,4 |
| S aureus | Klebsiella spp | S pneumoniae | H influenzae | | | 389,4 | 4 | 122,7 |
| Streptococcus spp | | | | | | 192,0 | 1 | 125,3 |
| Klebsiella spp | S Pneumoniae | H influenzae | Streptococcus spp | | | 396,6 | 4 | 129,9 |
| S aureus | S pneumoniae | H influenzae | M catarrhalis | | | 403,4 | 4 | 136,7 |
| Klebsiella spp | H influenzae | Streptococcus spp | M catarrhalis | | | 414,8 | 4 | 148,1 |
| H influenzae | Streptococcus spp | M catarrhalis | | | | 350,9 | 3 | 150,9 |
| S aureus | Klebsiella spp | H influenzae | Streptococcus spp | | | 420,3 | 4 | 153,6 |
| S aureus | S pneumoniae | Streptococcus spp | M catarrhalis | | | 425,2 | 4 | 158,5 |
| S pneumoniae | H influenzae | M catarrhalis | | | | 361,5 | 3 | 161,5 |
| S aureus | Klebsiella spp | S pneumoniae | M catarrhalis | | | 441,7 | 4 | 175,0 |
| S aureus | Klebsiella spp | H influenzae | | | | 388,8 | 3 | 188,8 |
| S aureus | Streptococcus spp | M catarrhalis | | | | 398,2 | 3 | 198,2 |
| Klebsiella spp | Streptococcus spp | | | | | 334,9 | 2 | 201,6 |
| Klebsiella spp | S Pneumoniae | | | | | 346,8 | 2 | 213,5 |
| Klebsiella spp | S pneumoniae | M catarrhalis | | | | 414,1 | 3 | 214,1 |
| S pneumoniae | Streptococcus spp | M catarrhalis | | | | 416,8 | 3 | 216,8 |
| S aureus | S pneumoniae | M catarrhalis | | | | 419,2 | 3 | 219,2 |
| H influenzae | | | | | | 290,0 | 1 | 223,3 |
| Klebsiella spp | S pneumoniae | Streptococcus spp | | | | 447,9 | 3 | 247,9 |
| H influenzae | M catarrhalis | | | | | 383,4 | 2 | 250,1 |
| Klebsiella spp | S pneumoniae | H influenzae | | | | 450,5 | 3 | 250,5 |
| Klebsiella spp | M catarrhalis | | | | | 399,9 | 2 | 266,6 |
| S aureus | S pneumoniae | H influenzae | | | | 476,6 | 3 | 276,6 |
| S pneumoniae | H influenzae | | | | | 470,7 | 2 | 337,4 |
| Streptococcus spp | M catarrhalis | | | | | 488,8 | 2 | 355,5 |
| Klebsiella spp | | | | | | 458,0 | 1 | 391,3 |
| M catarrhalis | | | | | | 463,0 | 1 | 396,3 |
| S pneumoniae | M catarrhalis | | | | | 531,0 | 2 | 397,7 |
| S aureus | M catarrhalis | | | | | 533,4 | 2 | 400,1 |

## Claims

1. A method of selecting bacteria or combination of bacteria suitable for preparing immunocompositions comprising the particulate fraction of lysates obtained by mechanical lysis of such bacteria for patient-customized treatments comprising the steps of:
selecting bacteria or combination of bacteria from the following genera *Staphylococcus, Klebsiella, Diplococcus, Haemophylous, Streptococcus, Neisseria,*
determining the expression level of the four membrane markers DC80, DC83, DC86 and HLA-II induced by each bacterium or combination of bacteria in immature dendritic cells;
calculating for each bacterium or combination of bacteria a cumulative score that is the sum of the amounts of the four expressed markers,
listing all bacteria or combination of bacteria by increasing cumulative score, wherein the higher the cumulative scores the higher the immunostimulation effect.

2. The method of claim 1, wherein the bacteria or combinations of bacteria are selected from the species: *Klebsiella spp, Haemophylous influenzae, Neisseria catarrhalis, Staphylococcus aureus, Diplococcus pneumoniae* and *Streptococcus spp.*

3. The method of claim 2, wherein at least one bacterium is selected from the species: *Klebsiella spp, Haemophylous influenzae* and *Neisseria catarrhalis* and the immunocomposition is an immunostimulating composition.

4. A method for preparing an immunocomposition comprising bacteria or combination of bacteria selected according to the method of any one of claims 1 to 3, wherein the bacterial cells or combinations of bacterial cells are mechanically lysed then the particulate fractions of the lysates are collected.

5. The method according to claim 4, wherein the particulate fractions of the lysates are collected and then freeze-dried.

6. The method according to claim 4, wherein the collected particulate fractions from each bacterium are freeze-dried before mixing.

7. The method according to claim 4, wherein the bacterial cells are formaldehyde or heat inactivated.

8. The method according to claims 4, wherein the particulate fraction is formulated into a pharmaceutical composition further comprising one or more of pharmaceutically acceptable carriers, excipients and/or additives.

9. The method according to any one of claims 4 to 8, wherein the bacteria or composition of bacteria are:
- *Staphylococcus aureus, Streptococcus spp;*
- *Staphylococcus aureus;*
- *Diplococcus pneumoniae, Streptococcus spp;*
- *Diplococcus pneumoniae;*
- *Staphylococcus aureus, Diplococcus pneumoniae;*
- *Diplococcus pneumoniae, Haemophylous influenzae, Streptococcus spp;*
- *Streptococcus spp;*
- *Staphylococcus aureus, Haemophylous influenzae;*
- *Staphylococcus aureus, Haemophylous influenzae, Streptococcus spp;*
- *Haemophylous influenzae, Streptococcus spp;*
- *Klebsiella spp, Haemophylous influenzae;*
- *Staphylococcus aureus, Klebsiella spp;*
- *Klebsiella spp, Haemophylous influenzae, Streptococcus spp;*
- *Staphylococcus aureus, Klebsiella spp, Streptococcus spp;*
- *Staphylococcus aureus, Diplococcus pneumoniae, Streptococcus spp;*
- *Klebsiella spp, Streptococcus spp, Neisseria catarrhalis;*
- *Staphylococcus aureus, Klebsiella spp, Neisseria catarrhalis;*
- *Klebsiella spp, Diplococcus pneumoniae, Streptococcus spp, Neisseria catarrhalis;*
- *Haemophylous influenzae;*
- *Klebsiella spp, Haemophylous influenzae, Neisseria catarrhalis;*
- *Staphylococcus aureus, Klebsiella spp, Diplococcus pneumoniae;*
- *Staphylococcus aureus, Haemophylous influenzae, Neisseria catarrhalis;*
- *Klebsiella spp, Streptococcus spp;*
- *Staphylococcus aureus, Haemophylous influenzae, Streptococcus spp, Neisseria catarrhalis;*
- *Klebsiella spp, Diplococcus pneumoniae;*
- *Klebsiella spp, Diplococcus pneumoniae, Haemophylous influenzae, Neisseria catarrhalis;*
- *Haemophylous influenzae, Streptococcus spp, Neisseria catarrhalis;*
- *Diplococcus pneumoniae, Haemophylous influenzae, Neisseria catarrhalis;*
- *Staphylococcus aureus, Klebsiella spp, Haemophylous influenzae, Neisseria catarrhalis;*
- *Staphylococcus aureus, Klebsiella spp, Diplococcus pneumoniae, Haemophylous influenzae, Streptococcus spp;*
- *Staphylococcus aureus, Klebsiella spp, Diplococcus pneumoniae, Streptococcus spp;*
- *Klebsiella spp, Diplococcus pneumoniae, Haemophylous influenzae, Streptococcus spp, Neisseria catarrhalis;*
- *Staphylococcus aureus, Diplococcus pneumoniae, Haemophylous influenzae, Streptococcus spp;*
- *Staphylococcus aureus, Klebsiella spp, Streptococcus spp, Neisseria catarrhalis;*
- *Haemophylous influenzae, Neisseria catarrhalis;*
- *Diplococcus pneumoniae, Haemophylous influenzae, Streptococcus spp, Neisseria catarrhalis;*
- *Staphylococcus aureus, Klebsiella spp, Haemophylous influenzae;*
- *Staphylococcus aureus, Klebsiella spp, Diplococcus pneumoniae, Haemophylous influenzae;*
- *Klebsiella spp, Diplococcus pneumoniae, Haemophylous influenzae, Streptococcus spp;*
- *Staphylococcus aureus, Diplococcus pneumoniae, Neisseria catarrhalis;*
- *Klebsiella spp, Neisseria catarrhalis;*
- *Staphylococcus aureus, Diplococcus pneumoniae, Haemophylous influenzae, Neisseria catarrhalis;*
- *Staphylococcus aureus, Klebsiella spp, Diplococcus pneumoniae, Haemophylous influenzae, Neisseria catarrhalis;*
- *Klebsiella spp, Diplococcus pneumoniae, Neisseria catarrhalis;*
- *Klebsiella spp, Haemophylous influenzae, Streptococcus spp, Neisseria catarrhalis;*
- *Diplococcus pneumoniae, Streptococcus spp, Neisseria catarrhalis;*
- *Staphylococcus aureus, Diplococcus pneumoniae, Neisseria catarrhalis;*
- *Staphylococcus aureus, Klebsiella spp, Haemophylous influenzae, Streptococcus spp;*
- *Staphylococcus aureus, Diplococcus pneumoniae, Streptococcus spp, Neisseria catarrhalis;*
- *Staphylococcus aureus, Klebsiella spp, Haemophylous influenzae, Streptococcus spp, Neisseria catarrhalis;*
- *Staphylococcus aureus, Klebsiella spp, Diplococcus pneumoniae, Neisseria catarrhalis;*
- *Klebsiella spp, Diplococcus pneumoniae, Streptococcus spp;*
- *Diplococcus pneumoniae, Staphylococcus aureus, Haemophylous influenzae, Streptococcus spp, Neisseria catarrhalis;*
- *Klebsiella spp, Diplococcus pneumoniae, Haemophylous influenzae;*
- *Klebsiella spp;*
- *Diplococcus pneumoniae, Haemophylous influenzae;*
- *Staphylococcus aureus, Diplococcus pneumoniae, Haemophylous influenzae;*
- *Streptococcus spp, Neisseria catarrhalis;*
- *Diplococcus pneumoniae, Neisseria catarrhalis* and
- *Staphylococcus aureus, Neisseria catarrhalis.*

10. A bacteria particulate immunocomposition for use in a medical immunotreatment, wherein the bacteria particulate is a fraction of lysates obtained by mechanical lysis of bacteria or combinations of bacteria selected from the group consisting of:
- *Staphylococcus aureus, Diplococcus pneumoniae, Haemophylous influenzae, Neisseria catarrhalis;*
- *Staphylococcus aureus, Klebsiella spp, Diplococcus pneumoniae, Haemophylous influenzae, Neisseria catarrhalis;*
- *Klebsiella spp, Diplococcus pneumoniae, Neisseria catarrhalis;*
- *Klebsiella spp, Haemophylous influenzae, Streptococcus spp, Neisseria catarrhalis;*
- *Diplococcus pneumoniae, Streptococcus spp, Neisseria catarrhalis;*
- *Staphylococcus aureus, Diplococcus pneumoniae, Neisseria catarrhalis;*
- *Staphylococcus aureus, Klebsiella spp, Haemophylous influenzae, Streptococcus spp;*
- *Staphylococcus aureus, Diplococcus pneumoniae, Streptococcus spp, Neisseria catarrhalis;*
- *Staphylococcus aureus, Klebsiella spp, Haemophylous influenzae, Streptococcus spp, Neisseria catarrhalis;*
- *Staphylococcus aureus, Klebsiella spp, Diplococcus pneumoniae, Neisseria catarrhalis;*
- *Klebsiella spp, Diplococcus pneumoniae, Streptococcus spp;*
- *Diplococcus pneumoniae, Staphylococcus aureus, Haemophylous influenzae, Streptococcus spp, Neisseria catarrhalis;*
- *Klebsiella spp, Diplococcus pneumoniae, Haemophylous influenzae;*
- *Klebsiella spp;*
- *Diplococcus pneumoniae, Haemophylous influenzae;*
- *Staphylococcus aureus, Diplococcus pneumoniae, Haemophylous influenzae;*
- *Streptococcus spp, Neisseria catarrhalis;*
- *Diplococcus pneumoniae, Neisseria catarrhalis* and
- *Staphylococcus aureus, Neisseria catarrhalis.*

11. The immunocomposition of claim 10 for use as a specific or aspecific immunomodulating agent.

12. Composition of claim 10 for use in the aspecific stimulation of the immunosystem, aspecific immunopotentiation upon vaccination, specific immunoprophylaxis for infections caused by bacterial strains contained in the composition, immunopotentiation of a vaccine effect in "non responder" patients, aspecific immunotherapy of tumours; aspecific immunotherapy of melanoma, bladder superficial tumour, NSCLC, specific immunotherapy of tumours with tumour-associated antigens of different molecular weight or tumour-associated lymphoid effectors or loaded dendritic cells, activation of the macrophage system as a scavenger in chronic diseases, decubitus or phagedenic ulcers, and tumours of the craniocephalic district.

## Patentansprüche

1. Verfahren zur Auswahl von Bakterien oder Kombinationen von Bakterien, welche geeignet sind zur Herstellung von Immunzusammensetzungen, umfassend eine Partikelfraktion von Lysaten, welche durch mechanische Lyse von solchen Bakterien erhalten wurde, zur Patienten-spezifischen Behandlung, umfassend die Schritte:
Auswahl von Bakterien oder Kombinationen von Bakterien von den folgenden Gattungen *Staphylococcus, Klebsiella, Diplococcus, Haemophylous, Streptococcus, Neisseria;*
Bestimmung des Expressionsspiegels der vier Membranmarker DC80, DC83, DC86 und HLA-II, welche durch jede(s) Bakterium oder Kombination von Bakterien in unreifen dendritischen Zellen induziert werden;
Berechnung eines kumulativen Werts für jede(s) Bakterium oder Kombination von Bakterien, welcher die Summe des Gehaltes der vier exprimierten Marker ist;
Auflistung aller Bakterien oder Kombination von Bakterien nach ansteigendem kumulativen Wert, wobei gilt: je höher die kumulativen Werte sind, desto höher ist der immunstimulierende Effekt.

2. Verfahren nach Anspruch 1, wobei die Bakterien oder Kombinationen von Bakterien ausgewählt sind aus den Spezies: *Klebsiella spp, Haemophylous influenza, Neisseria catarrhalis, Staphylococcus aureus, Diplococcus pneumoniae* und *Streptococcus spp.*

3. Verfahren nach Anspruch 2, wobei zumindest ein Bakterium ausgewählt ist aus den Spezies: *Klebsiella spp, Haemophylous influenzae,* und *Neisseria catarrhalis,* und die Immunzusammensetzung eine immunstimulierende Zusammensetzung ist.

4. Verfahren zur Herstellung einer Immunzusammensetzung, umfassend Bakterien oder Kombinationen von Bakterien ausgewählt gemäß dem Verfahren nach einem der Ansprüche 1 bis 3, wobei die Bakterienzellen oder Kombinationen von Bakterienzellen mechanisch lysiert und dann die Partikelfraktionen von den Lysaten gesammelt werden.

5. Verfahren gemäß Anspruch 4, wobei die Partikelfraktionen der Lysate gesammelt und dann gefriergetrocknet werden.

6. Verfahren gemäß Anspruch 4, wobei die gesammelten Partikelfraktionen von jedem Bakterium vor der Durchmischung gefriergetrocknet werden.

7. Verfahren gemäß Anspruch 4, wobei die Bakterienzellen mit Formaldehyd oder Hitze inaktiviert werden.

8. Verfahren gemäß Anspruch 4, wobei die Partikelfraktion als ein Arzneimittel formuliert wird, welches ferner einen oder mehrere pharmazeutisch verträgliche Träger, Exzipienten und/oder Zusatzstoffe umfasst.

9. Verfahren gemäß einem der Ansprüche 4 bis 8, wobei die Bakterien oder Bakterienzusammensetzung sind:
- *Staphylococcus aureus, Streptococcus spp;*
- *Staphylococcus aureus;*
- *Diplococcus pneumoniae, Streptococcus spp;*
- *Diplococcus pneumoniae;*
- *Staphylococcus aureus, Diplococcus pneumoniae;*
- *Diplococcus pneumoniae, Haemophylous influenzae, Streptococcus spp;*
- *Streptococcus spp;*
- *Staphylococcus aureus, Haemophylous influenzae;*
- *Staphylococcus aureus, Haemophylous influenzae, Streptococcus spp;*
- *Haemophylous influenzae, Streptococcus spp;*
- *Klebsiella spp, Haemophylous influenzae;*
- *Staphylococcus aureus, Klebsiella spp;*
- *Klebsiella spp, Haemophylous influenzae, Streptococcus spp;*
- *Staphylococcus aureus, Klebsiella spp, Streptococcus spp;*
- *Staphylococcus aureus, Diplococcus pneumoniae, Streptococcus spp;*
- *Klebsiella spp, Streptococcus spp, Neisseria catarrhalis;*
- *Staphylococcus aureus, Klebsiella spp, Neisseria catarrhalis;*
- *Klebsiella spp, Diplococcus pneumoniae, Streptococcus spp, Neiserria catarrhalis;*
- *Haemophylous influenzae;*
- *Klebsiella spp, Haemophylous influenzae, Neisseria catarrhalis;*
- *Staphylococcus aureus, Klebsiella spp, Diplococcus pneumoniae;*
- *Staphylococcus aureus, Haemophylous influenzae, Neisseria catarrhalis;*
- *Klebsiella spp, Streptococcus spp;*
- *Staphylococcus aureus, Haemophylous influenzae, Streptococcus spp, Neisseria catarrhalis;*
- *Klebsiella spp, Diplococcus pneumoniae;*
- *Klebsiella spp, Diplococcus pneumoniae, Haemophylous influenzae, Neisseria catarrhalis;*
- *Haemophylous influenzae, Streptococcus spp, Neisseria catarrhalis;*
- *Diplococcus pneumoniae, Haemophylous influenzae, Neisseria catarrhalis;*
- *Staphylococcus aureus, Klebsiella spp, Haemophylous influenzae, Neissaria catarrhalis;*
- *Staphylococcus aureus, Klebsiella spp, Diplococcus pneumoniae, Haemophylous influenzae, Streptococcus spp;*
- *Staphylococcus aureus, Klebsiella spp, Diplococcus pneumoniae, Streptococcus spp;*
- *Klebsiella spp, Diplococcus pneumoniae, Haemophylous influenzae, Streptococcus spp, Neisseria catarrhalis;*
- *Staphylococcus aureus, Diplococcus pneumoniae, Haemophylous influenzae, Streptococcus spp;*
- *Staphylococcus aureus, Klebsiella spp, Streptococcus spp, Neisseria catarrhalis;*
- *Haemophylous influenzae, Neisseria catarrhalis;*
- *Diplococcus pneumoniae, Haemophylous influenzae, Streptococcus ssp, Neisseria catarrhalis;*
- *Staphylococcus aureus, Klebsiella spp, Haemophylous influenzae;*
- *Staphylococcus aureus, Klebsiella spp, Diplococcus pneumoniae, Haemophylous influenzae;*
- *Klebsiella spp, Diplococcus pneumoniae, Haemophylous influenzae, Streptococcus spp;*
- *Staphylococcus aureus, Diplococcus pneumonia, Neisseria catarrhalis;*
- *Klebsiella spp, Neisseria catarrhalis;*
- *Staphylococcus aureus, Diplococcus pneumoniae, Haemophylous influenzae, Neisseria catarrhalis;*
- *Staphylococcus aureus, Klebsiella spp, Diplococcus pneumoniae, Haemophylous influenzae, Neisseria catarrhalis;*
- *Klebsiella spp, Diplococcus pneumoniae, Neisseria catarrhalis;*
- *Klebsiella spp, Haemophylous influenzae, Streptococcus spp, Neisseria catarrhalis;*
- *Diplococcus pneumoniae, Streptococcus spp, Neisseria catarrhalis;*
- *Staphylococcus aureus, Diplococcus pneumoniae, Neisseria catarrhalis;*
- *Staphylococcus aureus, Klebsiella spp, Haemophylous influenzae, Streptococcus spp;*
- *Staphylococcus aureus, Diplococcus pneumoniae, Streptococcus spp, Neisseria catarrhalis;*
- *Staphylococcus aureus, Klebsiella spp, Haemophylous influenzae, Streptococcus spp, Neisseria catarrhalis;*
- *Staphylococcus aureus, Klebsiella spp, Diplococcus pneumoniae, Neisseria catarrhalis;*
- *Klebsiella spp, Diplococcus pneumoniae, Streptococcus spp;*
- *Diplococcus pneumoniae, Staphylococcus aureus, Haemophylous influenzae, Streptococcus spp, Neisseria catarrhalis;*
- *Klebsiella spp, Diplococcus pneumoniae, Haemophylous influenzae;*
- *Klebsiella spp;*
- *Diplococcus pneumoniae, Haemophylous influenzae;*
- *Staphylococcus aureus, Diplococcus pneumoniae, Haemophylous influenzae;*
- *Streptococcus spp, Neisseria catarrhalis;*
- *Diplococcus pneumoniae, Neisseria catarrhalis;* und
- *Staphylococcus aureus, Neisseria catarrhalis.*

10. Immunzusammensetzung aus Bakterienpartikeln zur Verwendung in einer medizinischen Immunbehandlung, wobei die Bakterienpartikel eine Fraktion von Lysaten sind, welche durch mechanische Lyse von Bakterien oder Kombinationen von Bakterien erhalten wurde, welche ausgewählt sind aus der Gruppe bestehend aus:
- *Staphylococcus aureus, Diplococcus pneumoniae, Haemophylous influenzae, Neisseria catarrhalis;*
- *Staphylococcus aureus, Klebsiella spp, Diplococcus pneumoniae, Haemophylous influenzae, Neisseria catarrhalis;*
- *Klebsiella spp, Diplococcus pneumoniae, Neisseria catarrhalis;*
- *Klebsiella spp, Haemophylous influenzae,* Streptococcus spp, *Neisseria catarrhalis;*
- *Diplococcus pneumoniae, Streptococcus spp, Neisseria catarrhalis;*
- *Staphylococcus aureus, Diplococcus pneumoniae, Neisseria catarrhalis;*
- *Staphylococcus aureus, Klebsiella spp, Haemophylous influenzae, Streptococcus spp;*
- *Staphylococcus aureus, Diplococcus pneumoniae,* Streptococcus spp, *Neisseria catarrhalis;*
- *Staphylococcus aureus, Klebsiella spp, Haemophylous influenzae, Streptococcus spp, Neisseria catarrhalis;*
- *Staphylococcus aureus, Klebsiella spp, Diplococcus pneumoniae, Neisseria catarrhalis;*
- *Klebsiella spp, Diplococcus pneumoniae, Streptococcus spp;*
- *Diplococcus pneumoniae, Staphylococcus aureus, Haemophylous influenzae, Streptococcus spp, Neisseria catarrhalis;*
- *Klebsiella spp, Diplococcus pneumoniae, Haemophylous influenzae;*
- *Klebsiella spp;*
- *Diplococcus pneumoniae, Haemophylous influenzae;*
- *Staphylococcus aureus, Diplococcus pneumoniae, Haemophylous influenzae;*
- *Streptococcus spp, Neisseria catarrhalis;*
- *Diplococcus pneumoniae, Neisseria catarrhalis;* und
- *Staphylococcus aureus, Neisseria catarrhalis.*

11. Immunzusammensetzung nach Anspruch 10 zur Verwendung als ein spezifisches oder unspezifisches immunomodulierendes Mittel.

12. Zusammensetzung nach Anspruch 10 zur Verwendung in der unspezifischen Stimulierung des Immunsystems, unspezifischen Immunverstärkung nach Impfung, spezifischen Immunprophylaxe von Infektionen, welche durch die Infektion durch Bakterienstämme, welche in der Zusammensetzung enthalten sind, verursacht werden, Immunpotenzierung eines Impfeffekts in Non-Responder-Patienten, unspezfischen Immuntherapie von Tumoren, unspezifischen Immuntherapie von Melanomen, oberflächlichem Blasentumor, NSCLC, spezifischen Immuntherapie von Tumoren mit Tumor-assoziierten Antigenen von unterschiedlichem Molekulargewicht oder Tumor-assoziierten Lymphoideffektoren oder beladenen dendritischen Zellen, Aktivierung des Makrophagensystems als ein Scavenger bei chronischen Krankheiten, Druckgeschwüren oder sich schnell ausbreitendem Geschwüren und Tumoren des den Schädel betreffenden Bereichs.

## Revendications

1. Procédé de sélection de bactéries ou d'une combinaison de bactéries appropriées pour préparer des immunocompositions comprenant la fraction particulaire de lysats obtenus par lyse mécanique de telles bactéries pour des traitements adaptés aux patients comprenant les étapes de:
sélection de bactéries ou d'une combinaison de bactéries parmi les genres s suivants *Staphylococcus, Klebsiella, Diplococcus, Haemophylous, Streptococcus, Neisseria,*
détermination du niveau d'expression des quatre marqueurs membranaires DC80, DC83, DC86 et HLA-II induits par chaque bactérie ou combinaison de bactéries dans des cellules dendritiques immatures ;
calcul pour chaque bactérie ou combinaison de bactéries d'une note cumulative qui est la somme des quantités des quatre marqueurs exprimés,
établissement d'une liste de toutes les bactéries ou combinaison de bactéries par note cumulative croissante, où plus les notes cumulatives sont élevées, plus l'effet d'immunostimulation est élevé.

2. Procédé selon la revendication 1, où les bactéries ou combinaisons de bactéries sont choisies parmi les espèces: *Klebsiella spp, Haemophylous influenzae, Neisseria catarrhalis, Staphylococcus aureus, Diplococcus pneumoniae* et *Streptococcus spp.*

3. Procédé selon la revendication 2, où au moins une bactérie est choisie parmi les espèces: *Klebsiella spp, Haemophylous influenzae* et *Neisseria catarrhalis* et l'immunocomposition est une composition immunostimulante.

4. Procédé pour préparer une immunocomposition comprenant des bactéries ou une combinaison de bactéries choisies selon le procédé selon l'une quelconque des revendications 1 à 3, où les cellules bactériennes ou combinaisons de cellules bactériennes sont lysées mécaniquement puis les fractions particulaires des lysats sont recueillies.

5. Procédé selon la revendication 4, où les fractions particulaires des lysats sont recueillies puis lyophilisées.

6. Procédé selon la revendication 4, o ù les fractions particulaires recueillies à partir de chaque bactérie sont lyophilisées avant le mélange.

7. Procédé selon la revendication 4, où les cellules bactériennes sont inactivées par le formaldéhyde ou la chaleur.

8. Procédé selon la revendication 4, où la fraction particulaire est formulée dans une composition pharmaceutique comprenant en outre un ou plusieurs de vecteurs, excipients et/ou additifs pharmaceutiquement acceptables.

9. Procédé selon l'une quelconque des revendications 4 à 8, où les bactéries ou composition de bactéries sont:
- *Staphylococcus aureus, Streptococcus spp;*
- *Staphylococcus aureus;*
- *Diplococcus pneumoniae, Streptococcus spp;*
- *Diplococcus pneumoniae;*
- *Staphylococcus aureus, Diplococcus pneumoniae;*
- *Diplococcus pneumoniae, Haemophylous influenzae, Streptococcus spp;*
- *Streptococcus spp;*
- *Staphylococcus aureus, Haemophylous influenzae;*
- *Staphylococcus aureus, Haemophylous influenzae, Streptococcus spp;*
- *Haemophylous influenzae, Streptococcus spp;*
- *Klebsiella spp, Haemophylous influenzae;*
- *Staphylococcus aureus, Klebsiella spp;*
- *Klebsiella spp, Haemophylous influenzae, Streptococcus spp;*
- *Staphylococcus aureus, Klebsiella spp. Streptococcus spp;*
- *Staphylococcus aureus, Diplococcus pneumoniae, Streptococcus spp;*
- *Klebsiella spp, Streptococcus spp, Neisseria catarrhalis;*
- *Staphylococcus aureus, Klebsiella spp, Neisseria catarrhalis;*
- *Klebsiella spp, Diplococcus pneumonia, Streptococcus spp, Neisseria catarrhalis;*
- *Haemophylous influenzae;*
- *Klebsiella spp, Haemophylous influenzae, Neisseria catarrhalis;*
- *Staphylococcus aureus, Klebsiella spp, Diplococcus pneumoniae;*
- *Staphylococcus aureus, Haemophylous influenzae, Neisseria catarrhalis;*
- *Klebsiella spp, Streptococcus spp;*
- *Staphylococcus aureus, Haemophylous influenzae, Streptococcus spp, Neisseria catarrhalis;*
- *Klebsiella spp, Diplococcus pneumoniae;*
- *Klebsiella spp, Diplococcus pneumoniae, Haemophylous influenzae, Neisseria catarrhalis;*
- *Haemophylous influenzae, Streptococcus spp, Neisseria catarrhalis;*
- *Diplococcus pneumoniae, Haemophylous influenzae, Neisseria catarrhalis;*
- *Staphylococcus aureus, Klebsiella spp, Haemophylous influenzae, Neisseria catarrhalis;*
- *Staphylococcus aureus, Klebsiella spp, Diplococcus pneumoniae, Haemophylous influenzae, Streptococcus spp;*
- *Staphylococcus aureus, Klebsiella spp, Diplococcus pneumoniae, Streptococcus spp;*
- *Klebsiella spp, Diplococcus pneumoniae, Haemophylous influenzae, Streptococcus spp, Neisseria catarrhalis;*
- *Staphylococcus aureus, Diplococcus pneumoniae, Haemophylous influenzae, Streptococcus spp;*
- *Staphylococcus aureus, Klebsiella spp, Streptococcus spp, Neisseria catarrhalis;*
- *Haemophylous influenzae, Neisseria catarrhalis;*
- *Diplococcus pneumoniae, Haemophylous influenzae, Streptococcus spp, Neisseria catarrhalis;*
- *Staphylococcus aureus, Klebsiella spp, Haemophylous influenzae;*
- *Staphylococcus aureus, Klebsiella spp, Diplococcus pneumoniae, Haemophylous influenzae;*
- *Klebsiella spp, Diplococcus pneumoniae, Haemophylous influenzae, Streptococcus spp;*
- *Staphylococcus aureus, Diplococcus pneumoniae, Neisseria catarrhalis;*
- *Klebsiella spp, Neisseria catarrhalis;*
- *Staphylococcus aureus, Diplococcus pneumoniae, Haemophylous influenzae, Neisseria catarrhalis;*
- *Staphylococcus aureus, Klebsiella spp, Diplococcus pneumoniae, Haemophylous influenzae, Neisseria catarrhalis;*
- *Klebsiella spp, Diplococcus pneumoniae, Neisseria catarrhalis;*
- *Klebsiella spp, Haemophylous influenzae, Streptococcus spp, Neisseria catarrhalis;*
- *Diplococcus pneumoniae, Streptococcus spp, Neisseria catarrhalis;*
- *Staphylococcus aureus, Diplococcus pneumoniae, Neisseria catarrhalis;*
- *Staphylococcus aureus, Klebsiella spp, Haemophylous influenzae, Streptococcus spp;*
- *Staphylococcus aureus, Diplococcus pneumoniae, Streptococcus spp, Neisseria catarrhalis;*
- *Staphylococcus aureus, Klebsiella spp, Haemophylous influenzae, Streptococcus spp, Neisseria catarrhalis;*
- *Staphylococcus aureus, Klebsiella spp, Diplococcus pneumoniae, Neisseria catarrhalis;*
- *Klebsiella spp, Diplococcus pneumoniae, Streptococcus spp;*
- *Diplococcus pneumoniae, Staphylococcus aureus, Haemophylous influenzae, Streptococcus spp, Neisseria catarrhalis;*
- *Klebsiella spp, Diplococcus pneumoniae, Haemophylous influenzae;*
- *Klebsiella spp;*
- *Diplococcus pneumoniae, Haemophylous influenzae;*
- *Staphylococcus aureus, Diplococcus pneumoniae, Haemophylous influenzae;*
- *Streptococcus spp, Neisseria catarrhalis;*
- *Diplococcus pneumoniae, Neisseria catarrhalis* et
- *Staphylococcus aureus, Neisseria catarrhalis.*

10. Immunocomposition de particule de bactéries destinée à être utilisée dans un immunotraitement médical où la particule de bactéries est une fraction de lysats obtenue par lyse mécanique de bactéries ou combinaisons de bactéries choisies dans le groupe consistant en :
- *Staphylococcus aureus, Diplococcus pneumoniae, Haemophylous influenzae, Neisseria catarrhalis;*
- *Staphylococcus aureus, Klebsiella spp, Diplococcus pneumoniae, Haemophylous influenzae, Neisseria catarrhalis;*
- *Klebsiella spp, Diplococcus pneumoniae. Neisseria catarrhalis;*
- *Klebsiella spp, Haemophylous influenzae, Streptococcus spp, Neisseria catarrhalis;*
- *Diplococcus pneumoniae, Streptococcus spp, Neisseria catarrhalis;*
- *Staphylococcus aureus, Diplococcus pneumoniae, Neisseria catarrhalis;*
- *Staphylococcus aureus, Klebsiella spp, Haemophylous influenzae, Streptococcus spp;*
- *Staphylococcus aureus, Diplococcus pneumoniae, Streptococcus spp, Neisseria catarrhalis;*
- *Staphylococcus aureus, Klebsiella spp, Haemophylous influenzae, Streptococcus spp, Neisseria catarrhalis;*
- *Staphylococcus aureus, Klebsiella spp, Diplococcus pneumoniae, Neisseria catarrhalis;*
- *Klebsiella spp, Diplococcus pneumoniae, Streptococcus spp;*
- *Diplococcus pneumoniae, Staphylococcus aureus, Haemophylous influenzae, Streptococcus spp, Neisseria catarrhalis;*
- *Klebsiella spp, Diplococcus pneumoniae, Haemophylous influenzae;*
- *Klebsiella spp;*
- *Diplococcus pneumoniae, Haemophylous influenzae;*
- *Staphylococcus aureus, Diplococcus pneumoniae, Haemophylous influenzae;*
- *Streptococcus spp, Neisseria catarrhalis;*
- *Diplococcus pneumoniae, Neisseria catarrhalis* et
- *Staphylococcus aureus, Neisseria catarrhalis.*

11. Immunocomposition selon la revendication 10 destinée à être utilisée comme agent immunomodulateur spécifique ou aspécifique.

12. Composition selon la revendication 10 destinée à être utilisée dans la stimulation aspécifique du système immunitaire, l'immunostimulation aspécifique lors d'une vaccination, l'immunoprophylaxie spécifique pour les infections causées par des souches bactériennes contenues dans la composition, l'immunostimulation de l'effet d'un vaccin chez des patients « non répondeurs », l'immunothérapie aspécifique de tumeurs; l'immunothérapie aspécifique du mélanome, d'une tumeur superficielle de la vessie, du NSCLC, l'immunothérapie spécifique de tumeurs avec des antigènes associés aux tumeurs de différente masse moléculaire ou des effecteurs lymphoïdes associés aux tumeurs ou des cellules dendritiques chargées, l'activation du système des macrophages comme épurateur dans les maladies chroniques, les ulcères de décubitus ou phagédéniques, et les tumeurs du district craniocéphalique.
